(19) Europäisches Patentamt
European Patent Office
Office européen des brevets

(11) Veröffentlichungsnummer: **0 288 072**
**A2**

(12) # EUROPÄISCHE PATENTANMELDUNG

(21) Anmeldenummer: **88106488.5**

(51) Int. Cl.⁴: **A61K 31/47 , C07D 215/38**

(22) Anmeldetag: **22.04.88**

Claims for the following Contracting States: ES + GR.

(30) Priorität: **24.04.87 HU 177787**

(43) Veröffentlichungstag der Anmeldung: **26.10.88 Patentblatt 88/43**

(84) Benannte Vertragsstaaten: **AT BE CH DE ES FR GB GR IT LI NL SE**

(71) Anmelder: **EGIS Gyogyszergyar**
**Kereszturi ut 30-38**
**Budapest X(HU)**

(72) Erfinder: **Knoll, József, Dr.**
**Jászai M. tér 4/b**
**Budapest XIII(HU)**
Erfinder: **Budai geb. Simonyi, Katalin, Dr.**
**Lukács u. 3**
**Budapest II(HU)**
Erfinder: **Berényi geb. Poldermann, Edit, Dr.**
**Bartók B. u. 36-38**
**Budapest XI(HU)**
Erfinder: **Miklya, Ildikó**
**Mezö I., u. 21-23**
**Budapest VIII(HU)**
Erfinder: **Fekete, Márton, Dr.**
**Fö U. 49**
**H-1027 Budapest(HU)**
Erfinder: **Zsilla, Gabriella, Dr.**
**Vörös H. u. 299**
**Budapest XVIII(HU)**
Erfinder: **Knoll, Berta, Dr.**
**Jászai M. tér 4/b**
**Budapest XIII(HU)**
Erfinder: **Mándi, Attila, Dr.**
**Endrödi S. u. 7/8**
**Budapest(HU)**
Erfinder: **Petöcz, Lujza, Dr.**
**Rákóczi tér 2**
**Budapest VIII(HU)**
Erfinder: **Gyertyán, István**
**Összefogás sétány 9**
**H-1165 Budapest(HU)**
Erfinder: **Gacsályi, István**
**Baross u. 67**
**H-1201 Budapest(HU)**

(74) Vertreter: **Beszédes, Stephan G., Dr.**
**Münchener Strasse 80a Postfach 1168**
**D-8060 Dachau(DE)**

(54) **3-(Amino)-4-äthylthio)-chinolin oder dessen Säureadditionssalze zur Verwendung als Arzneimittel und diese[s] enthaltende Arzneimittel sowie Verfahren zur Herstellung des beziehungsweise der ersteren.**

EP 0 288 072 A2

Xerox Copy Centre

(57) Gegenstand der Erfindung sind 3-(Amino)-5-(äthylthio)-chinolin der Formel

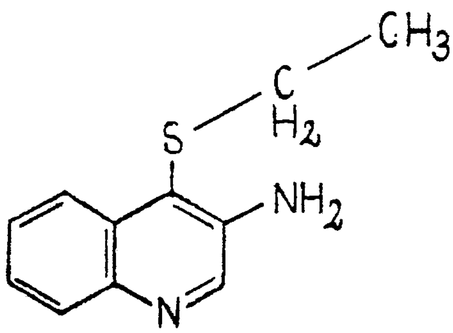

I

oder desen Säureadditionssalze zur Verwendung als Arzneimittel.

Gegenstand der Erfindung ist auch ein Verfahren zur Herstellung des beziehungsweise der 3-(Amino)-4-(äthylthio)-chinolines beziehungsweise Säureadditionssalze desselben sowie diese[s] enthaltende Arzneimittel.

# 3-(Amino)-4-(äthylthio)-chinolin oder dessen Säureadditionssalze zur Verwendung als Arzneimittel und diese[s] enthaltende Arzneimittel sowie Verfahren zur Herstellung des beziehungsweise der ersteren

Die Erfindung betrifft 3-(Amino)-4-(äthylthio)-chinolin oder dessen Säureadditionssalze zur Verwendung als Arzneimittel und diese[s] enthaltende Arzneimittel sowie ein Verfahren zur Herstellung des beziehungsweise der ersteren.

Das 3-(Amino)-(äthylthio)-chinolin ist im Schrifttum beschrieben (Hiroyuki Sawanishi und Mitarbeiter: "Heterocycless" 22/7 [1984], 1 501 bis 1 504). Nach diesem Artikel wird diese Verbindung in der Weise hergestellt, daß 3-(Azido)-chinolin in Gegenwart von Äthanthiol einer Photolyse oder Thermolyse unterworfen wird. In dieser Druckschrift wird jedoch über die biologische Wirkung des 3-(Amino)-4-(äthylthio)-chinolines nichts gesagt.

Das in der obigen Druckschrift beschriebene Verfahren ist mit mehreren Nachteilen verbunden. Es spielt nur eine theoretische Rolle und ist im Betriebsmaßstab kaum durchführbar. Die photochemische Reaktion kann nämlich nur in kleinem Maßstab und in spezifischen Einrichtungen verwirklicht werden. Ferner ist der Ausgangsstoff 3-(Azido)-chinolin sehr explosionsgefährlich und der Reaktionsteilnehmer Äthanthiol sehr toxisch.

Der Erfindung liegt die Aufgabe zugrunde, das 3-(Amino)-4-(äthylthio)-chinolin beziehungsweise dessen Säureadditionssalze für eine neue Verwendung und sowie unter Behebung der Nachteile der bekannten Verfahren ein besseres Verfahren zur Herstellung des beziehungsweise der ersteren, welches auch im Betriebsmaßstab einfach durchführbar ist, und das 3-(Amino)-4-(äthylthio)-chinolin beziehungsweise dessen Säureadditionssalze enthaltende Arzneimittel, zu schaffen.

Dies wurde überraschenderweise durch die Erfindung erreicht. Es wurde nämlich nun überraschenderweise festgestellt, daß das 3-(Amino)-4-(äthylthio)-chinolin und dessen Säureadditionssalze wertvolle pharmazeutische Eigenschaften, insbesondere eine starke anxiolytische und narkosepotenzierende Wirkung, haben.

Gegenstand der Erfindung ist daher das 3-(Amino)-4-(äthylthio)-chinolin der Formel

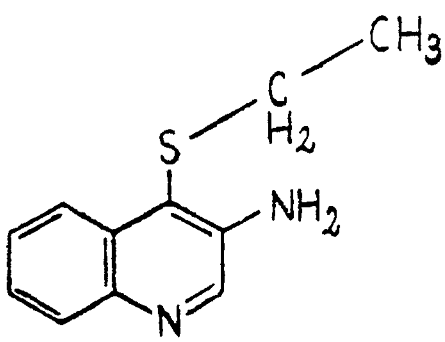

I

oder dessen Säureadditionssalze zur Verwendung als Arzneimittel.

Vorzugsweise sind die Arzneimittel, für welche das 3-(Amino)-4-(äthylthio)-chinolin beziehungsweise dessen Säureadditionssalze bestimmt ist beziehungsweise sind, solche mit anxiolytischer und/oder narkosepotenzierender Wirkung.

Es ist auch bevorzugt, daß das Säureadditionssalz von 3-(Amino)-4-(äthylthio)-chinolin 3-(Amino)-4-(äthylthio)-chinolinhydrochlorid ist.

Der Vorteil des 3-(Amino)-4-(äthylthio)-chinolines beziehungsweise seiner Säureadditionssalze liegt darin, daß es beziehungsweise sie eine sehr hohe anxiolytische Selektivität aufweist beziehungsweise aufweisen, das heißt von sedativen und krampfhemmenden (antikonvulsiven) Wirkungen praktisch frei ist beziehungsweise sind.

Die pharmakologischen Wirkungen des 3-(Amino)-4-(äthylthio)-chinolines wurden an Hand der folgenden Prüfversuche nachgewiesen.

Zu den Versuchen wurde das 3-(Amino)-4-(äthylthio)-chinolin-hydrochlorid verwendet.

Das 3-(Amino)-4-(äthylthio)-chinolin-hydrochlorid hat die folgende Toxizität:

oral $LD_{50}$-Wert (an Ratten) 420 mg/kg;

subkutan (s.c.) $LD_{50}$-Wert (an Ratten): 320 mg/kg.

Das 3-(Amino)-4-(äthylthio)-chinolin-hydrochlorid zeigt die folgende Aktivität im wohlbekannten "Heißplatten"-Prüfungsversuch ["hot plate"-Test]:

subkutan (s.c.) $ED_{50}$-Wert = 40 mg/kg;
oral $ED_{50}$-Wert = 49 mg/kg.

Das 3-(Amino)-4-(äthylthio)-chinolin-hydrochlorid zeigt im algolytischen Prüfversuch keine analgetische Wirksamkeit (an Ratten).

Das 3-(Amino)-4-(äthylthio)-chinolin-hydrochlorid zeigt an Ratten bei Verwendung von Äthyl-(1-methyl-propyl)-thiobarbiturat - Natrium [Inactin] eine sehr starke narkosepotenzierende Wirkung. Die Dosis, welche die Wirkungsdauer der Äthyl-(1-methyl-propyl)-thiobarbiturat - Natrium-Narkose [Inactinnarkose] auf das 5-fache erhöht, ist wie folgt: $ED_{500}$-Wert = 3,5 mg/kg subkutan (s.c.) und 3,4 mg/kg peroral (p.o.). Auf Grund der Tatsache, daß das 3-(Amino)-4-(äthylthio)-chinolin-hydrochlorid die Wirkung der 5-(1-Cyclohexenyl)-1,5-dimethylbarbitursäure [des Hexobarbitals] viel schwächer und die Aktivität eines von den Barbituraten verschiedenen Phenyclidinderivates, des Calypsols, überhaupt nicht beeinflußt, wird die Verlängerung der Äthyl-(1-methyl-propyl)-thiobarbiturat - Natrium-Narkose [Inactinnarkose] wahrscheinlich durch eine metabolische Korrelation hervorgerufen, das heißt nicht durch eine allgemeine zentrale Wirkung verursacht.

Das 3-(Amino)-4-(äthylthio)-chinolin-hydrochlorid ist gegen durch Tetracor hervorgerufene Krämpfe in Dosen von 25 beziehungsweise 100 mg/kg peroral (p.o.) als Spasmolytikum unwirksam.

Im modifizierten Sprungtest hemmt das 3-(Amino)-4-(äthylthio)-chinolin-hydrochlorid den unbedingten Abwehrreflex in Dosen von 50 und 100 mg/kg peroral (p.o.) nicht.

Das 3-(Amino)-4-(äthylthio)-chinolin-hydrochlorid hemmt die spontane Motilität an Ratten in hohen Dosen von 50 und 100 mg/kg peroral (p.o.) mittelmäßig und nicht dosisabhängig. In einer Dosis von 25 mg/kg ist das 3-(Amino)-4-(äthylthio)-chinolin-hydrochlorid diesbezüglich unwirksam.

Im einrichtungskonditionierten bedingten Abwehrreaktionsprüfversuch (Screening Test I) zeigt das 3-(Amino)-4-(äthylthio)-chinolin-hydrochlorid auch in einer hohen Dosis von 100 mg/kg peroral (p.o.) nur eine sehr schwache hemmende Wirkung.

Im zweirichtungskonditionierten bedingten Abwehrreaktionsprüfversuch ["Shuttle-Box Test"] hemmt das 3-(Amino)-4-(äthylthio)-chinolin-hydrochlorid das Lernvermögen erst in der verhältnismäßig hohen Dosis von 100 mg/kg peroral (p.o.) signifikant.

Das 3-(Amino)-4-(äthylthio)-chinolin-hydrochlorid beeinflußt den Stoffwechsel an Ratten nicht einmal in einer Dosis von 100 mg/kg peroral (p.o.).

Die anxiolytische beziehungsweise sedative Wirkung wurde im nachstehenden Konfliktprüfversuch untersucht und die erhaltenen Ergebnisse sind in der folgenden Tabelle zusammengestellt. Die Messung der anxiolytischen Wirkung der zu untersuchenden Verbindung beruht auf dem Prinzip, daß die in Konfliktsituationen gezeigte Aktivität der zu untersuchenden Verbindung beobachtet wird. Es wird eine belohnte Reaktion zustandegebracht, wonach diese aufgetretene Reaktion durch eine Strafe zurückgedrängt wird. Die anxiolytische Wirkung wird auf Grund der Bestimmung der durch die zu untersuchende Verbindung hervorgerufenen Erhöhung der bestraften Reaktion gemessen. Als Maß der sedativen Wirkung dient die durch die zu untersuchende Verbindung verursachte Herabsetzung der unbetraften Reaktion.

Den Versuchratten wurde das Futter 96 Stunden lang entzogen; während dieser Zeitdauer erhielten jedoch die Tiere nach Belieben (ad libitum) Trinkwasser. Während der 4-tägigen Futterentziehungsperiode tranken die Tiere etwa 7 ml Wasser, wobei die Wasseraufnahme von Ratten, welche trockenes Futter erhielten, auf 35 ml/Tag stieg (siehe J. Knoll: J. Neural. Trans. 59 [1984], 163 bis 194).Dies bedeutet, daß, falls das Tränkrohr mit dem den Käfigboden bildenden Gitter elektrisch verbunden und dem Tier, welches zu trinken versucht, systematisch ein Schlag durch elektrischen Strom versetzt wird, die Ratte, welche zur Futtereinnahme ohne Trinken unfähig ist, auf eine maximale Weise gezwungen wird, das Hindernis aus dem Wege zu räumen. Dieser Prüfversuch ist eine sehr empfindliche Verfahrensweise zur Bestimmung der anxiolytischen Wirkung.

Im einzelnen wurde der Versuch wie folgt durchgeführt.

Männliche CFY-Ratten (Körpergewicht: 230 bis 250 g) wurden ständig an Standard-Futterpellets gehalten; die Tiere erhielten Leitungswasser nach Belieben. Die Tiere wurden in aus je 10 Ratten bestehenden Gruppen 2 Wochen lang unter Standard-Umgebungsbedingungen (bei 22 bis 24°C) bis zum Versuchsbeginn gehalten. Den in Käfigen einzeln gehaltenen Ratten wurde das Futter während der Hungerperiode 96 Stunden lang entzogen; vor dem Versuchsbeginn erhierlten die Tiere Leitungswasser nach Belieben.

Danach wurden für den Versuch diejenigen Tiere ausgewählt, welche in der Hungerperiode eine Körpergewichtsabnahme von nicht mehr als 80 g zeigten. Die Körpergewichtsabnahme der oben eingesetzten 550 männlichen CFY-Ratten betrug durchschnittlich 66 g.

Ein weiterer Gesichtspunkt der Tierauswahl liegt darin, daß die Tiere nach der Hungerperiode sichtbar einen guten physikalischen Zustand aufweisen und eine normale Aktivität zeigen sollen. Wegen eines ungenügenden physikalischen Zustandes mußten weniger als 10% der Tiere ausgeschlossen werden.

Zum Versuch wurde eine Schachtel (39 x 27 x 12 cm), welche aus durchsichtigem Plexiglas hergestellt und durch einen Gitterboden (für die Futterpellets) und ein Tränkrohr versehen war, verwendet. Der Gitterboden, das Tränkrohr und der elektrische Stimulator (Grass S48) wurden elektrisch verbunden und im Stromkreis wurden in Zeitintervallen von 20 Sekunden 10 Sekunden lang anhaltende elektrische Stromschläge zustandegebracht. Die Stromparameter (100 V; 25 Ω; 7,5 Sek.; 5 Hz) wurden empirisch so ausgewählt, daß die elektrischen Stromschläge bei den sehr hungrigen Ratten die Wasseraufnahme auf $\frac{1}{3}$ im Verhältnis zum Blindversuchswert (unbestraften Wert) herabsetzten.

Nach der 96-stündigen Futterentziehungsperiode verbrachten die Tiere 1 Stunde in der Vorrichtung. Die sehr hungrigen Ratten verzehrten nach der langen Hungerperiode in der ersten Stunde 5 bis 6 g Futterpellets. Die Wasseraufnahme der sehr hungrigen Ratten wurde in der ersten Stunde der Fütterungsperiode gemessen; in der unbestraften Rattengruppe betrug die Wasseraufnahme 6 bis 7 ml. Unter Einwirkung der Strafe wurde die Wasseraufnahme unter 3 ml herabgesetzt.

Die zu untersuchende Verbindung 3-(Amino)-4-(äthylthio)-chinolin-hydrochlorid wurde parenteral, und zwar speziell subkutan in einem Dosisvolumen von 0,5 ml/100 g Körpergewicht verabreicht. Wegen der langen Futterentziehungsperiode wurde von der oralen Wirkstoffverabreichung abgesehen.

Tabelle

| 3-(Amino)-4-(äthylthio)--chinolin-hydrochlorid | "Konflikt-Prüfversuch" | | | |
|---|---|---|---|---|
| | Mit Strom | | Ohne Strom | |
| Subkutane [s.c.] Dosis in 0,5 ml/100 g $\frac{1}{2}$ Stunde bzw. destilliertes Wasser [Blindversuch] | Wasseraufnahme in ml | Zahl der Tiere | Wasseraufnahme in ml | Zahl der Tiere |
| 2,5 γ/kg | 3.4 ± 0,43[x] | 10 | - | - |
| 5 γ/kg | 4,1 ± 0,66[xxx] | 10 | - | - |
| 10 γ/kg | 2,7 ± 0,33 | 10 | - | - |
| 25 γ/kg | 2,8 ± 0,38 | 10 | - | - |
| 0,25 mg/kg | 3,3 ± 0,45[x] | 10 | 6,1 ± 0,46 | 10 |
| 5 mg/kg | - | - | 7,2 ± 0,37 | 10 |
| 10 mg/kg | - | - | 6,9 ± 0,43 | 10 |
| 25 mg/kg | - | - | 3,4 ± 0,60[xx] | 7 |
| Blindversuch (destilliertes Wasser) subkutan [s.c.] 0,5 mg/100 g $\frac{1}{2}$ Stunde | 2,3 ± 0,19 | 40 | 6,4 ± 0,57 | 15 |

x $p < 0{,}05$
xx $p < 0{,}01$
xxx $p < 0{,}001$

Aus der obigen Tabelle geht hervor, daß das 3-(Amino)-4-(äthylthio)-chinolin-hydrochlorid die durch Stromschläge bestrafte Wasseraufnahme bereits in einer Dosis von 2,5 γ/kg subkutan (s.c.) signifikant erhöht, das heißt, daß das 3-(Amino)-4-(äthylthio)-chinolin-hydrochlorid eine äußerst wirksame anxiolytische Aktivität zeigt. Dagegen wird eine sedative Wirkung erst in einer Dosis von 25 mg/kg subkutan (s.c.) beobachtet. Das Verhältnis der anxiolytischen und sedativen Wirkungen beträgt beim 3-(Amino)-4-(äthylthio)-chinolin-hydrochlorid 25 000 : 2,5 = 10 000; der entsprechende Wert des 7-(Chlor)-2-(methylamino)-5-(phenyl)-3H-1,4-benzodiazepin-4-oxydes [Chlordiazepoxydes] ist im selben Prüversuch 10 : 0,1 = 100. Aus dem Obigen geht hervor, daß beim 3-(Amino)-4-(äthylthio)-chinolin-hydrochlorid die Dissoziation zwischen den sedativen und anxiolytischen Dosen äußerst groß ist.

Das 3-(Amino)-4-(äthylthio)-chinolin-hydrochlorid drängt das Diazepam von den Benzodiazepinrezeptoren hinaus.

Ferner wurde erfindungsgemäß überraschenderweise festgestellt, daß durch das im folgenden festgelegte Verfahren die der Erfindung zugrundeliegende Teilaufgabe, ein besseres Verfahren zur Herstellung des 3-(Amino)-4-(äthylthio)-chinolines beziehungsweise der Säureadditionssalze desselben, welches auch im Betriebsmaßstab durchführbar ist, zu schaffen, gelöst wurde.

Gegenstand der Erfindung ist daher auch ein Verfahren zur Herstellung des beziehungsweise der 3-(Amino)-4-(äthylthio)-chinolines beziehungsweise Säureadditionssalze desselben, welches dadurch gekennzeichnet ist, daß

a) 3-(Amino)-4-(mercapto)-chinolin der Formel

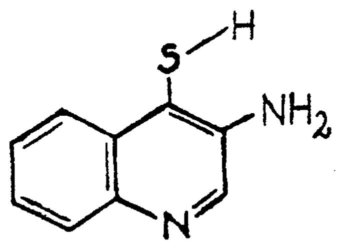

II

äthyliert wird oder

b) 3-(Nitro)-4-(mercapto)-chinolin der Formel

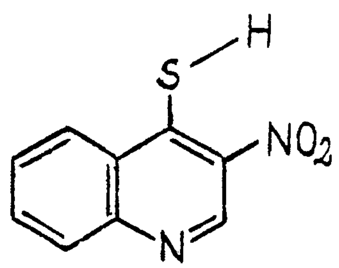

III

äthyliert und danach das erhaltene 3-(Nitro)-4-(äthylthio)-chinolin der Formel

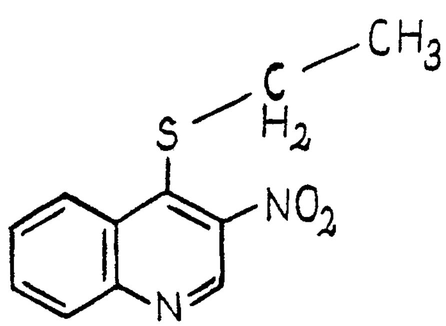

IV

reduziert wird oder

c) 3-(Nitro)-4-(chlor)-chinolin der Formel

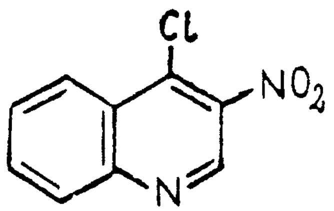

V

mit einem Alkaliäthanthiolat umgesetzt und das erhaltene 3-(Nitro)-4-(äthylthio)-chinolin der Formel IV reduziert wird, worauf in an sich bekannter Weise gegebenenfalls das erhaltene 3-(Amino)-4-(äthylthio)-chinolin der Formel

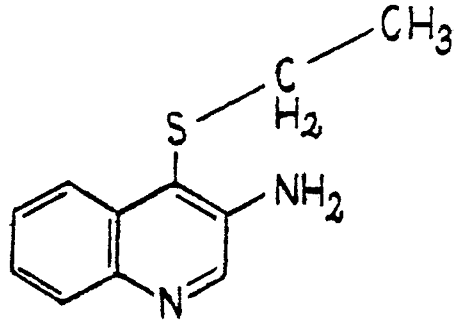

I

in ein Säureadditionssalz überfuhrt wird und/oder das erhaltene Säureadditionssalz des 3-(Amino)-4-(äthylthio)-chinolines der Formel I in die freie 3-(Amino)-4-(äthylthio)-chinolinbase oder in ein anderes Säureadditionssalz überführt wird.

Zum Äthylieren des 3-(Amino)-4-(mercapto)-chinolines der Formel II der Variante a) des erfindungsgemäßen Verfahrens können übliche Äthylierungsverfahrensweisen angewandt werden. Als Äthylierungsmittel kann beziehungsweise können vorteilhaft 1 oder mehr Äthylhalogenid(e), vorzugsweise Äthyljodid, Diäthylsulfat und/oder Arylsulfonsäureäthylester, vorzugsweise Benzolsulfonsäure, eingesetzt werden. Die Umsetzung wird zweckmäßig in Gegenwart von 1 oder mehr Base(n) durchgeführt. Insbesondere wird das Athylieren mit Diäthylsulfat oder Benzolsulfonsäureäthylester in Gegenwart von 1 oder mehr Alkalihydroxyd-(en), vorzugsweise von Natrium-und/oder Kaliumhydroxyd, durchgeführt. Als Reaktionstemperatur werden zwechmäßig 20 bis 160°C angewandt. Vorteilhaft wird unter Erwärmen gearbeitet. Vorzugsweise wird das Äthylieren beim Siedepunkt des Reaktionsgemisches durchgeführt. Zweckmäßig wird die Umsetzung in 1 oder mehr polaren Lösungsmittel(n), beispielsweise in einem wäßrigen Medium, durchgeführt. Das erhaltene 3-(Amino)-4-(äthylthio)-chinolin der Formel I kann aus dem Reaktionsgemisch durch bekannte Verfahrensweisen, zum Beispiel Filtrieren, Einengen oder Extrahieren, isoliert werden.

Auch zum Athylieren des 3-(Nitro)-4-(mercapto)-chinolines der Formel III der Variante b) des erfindungsgemäßen Verfahrens können übliche Äthylierungsverfahren angewandt werden. Auch hierfür gelten die vorstehend angegebenen bevorzugten, vorteilhaften und zweckmäßigen Merkmale sinngemäß.

Zum Reduzieren des in der ersten Stufe dieser Variante b) des erfindungsgemäßen Verfahrens erhaltenen 3-(Nitro)-4-(äthylthio)-chinolines der Formel IV können übliche Reduktionsverfahrensweisen angewandt werden. Die Umsetzung kann zweckmäßig mit Hilfe von 1 oder mehr Alkalisulfid(en), vorzugsweise Natriumsulfid, durchgeführt werden. Es ist auch zweckmäßig, in einem wäßrigen Medium zu arbeiten. Vorteilhaft wird unter Erwärmen, insbesondere unter Erhitzen zum Sieden unter Rückfluß gearbeitet. Das erhaltene 3-(Amino)-4-(äthylthio)-chinolin der Formel I kann aus dem Reaktionsgemisch auf bekannte Weise isoliert werden.

Zum Umsetzen des 3-(Nitro)-4-(chlor)-chinolines der Formel V mit dem Alkaliäthanthiolat der Variante c) des erfindungsgemäßen Verfahrens wird als das letztere vorzugsweise Natriumäthanthiolat verwendet. Vorteilhaft wird diese Umsetzung in einem organischen Lösungsmittel, gegebenenfalls in Gegenwart von Wasser, durchgeführt. Sie kann bei Raumtemperatur oder unter schwachem Erwärmen vorgenommen werden.

Zum Reduzieren des in der ersten Stufe dieser Variante c) des erfindungsgemäßen Verfahrens erhaltenen 3-(Nitro)-4-(äthylthio)-chinolines der Formel V können übliche Reduktionsverfahren angewandt werden. Auch hierfür gelten die für die Reduktion der Variante b) des erfindungsgemäßen Verfahrens angegebenen bevorzugten, vorteilhaften und zweckmäßigen Merkmale sinngemäß.

Das Überführen des erhaltenen 3-(Amino)-4-(äthylthio)-chinolines der Formel I in Säureadditionssalze kann mit pharmazeutisch brauchbaren anorganischen Säuren, zum Beispiel Salzsäure, Bromwasserstoffsäure, Schwefelsäure oder Phosphorsäure, oder starken pharmazeutisch brauchbaren organischen Säuren, zum Beispiel Äthansulfonsäure, durchgeführt werden. Die Salzbildung kann in an sich bekannter Weise durch Umsetzen der 3-(Amino)-4-(äthylthio)-chinolinbase der Formel I mit einer moläquivalenten Menge der entsprechenden Säure in 1 oder mehr inerten organischen Lösungsmittel(n) durchgeführt werden.

Die im erfindungsgemäßen Verfahren verwendeten Ausgangsstoffe der Formeln II bis IV sind teilweise bekannt (Bachman und Mitarbeiter: J. Am. Chem. Soc. 69 [1947], 365 bis 375) oder können analog zur

Herstellung von bekannten Verbindungen hergestellt worden sein.

Die Vorteile des erfindungsgemäßen Verfahrens sind zusammengefaßt wie folgt:

a) Das Verfahren kann auch im Betriebsmaßstab einfach und vorteilhaft durchgeführt werden.

b) Die Ausbeute ist hoch.

c) Die Ausgangsstoffe sind leicht zugänglich und mit geringem Aufwand verbunden.

Gegenstand der Erfindung sind auch Arzneimittel, welche durch einen Gehalt an 3-(Amino)-4-(äthylthio)-chinolin und/oder 1 oder mehr Säureadditionssalz(en) desselben als Wirkstoff(en) sowie 1 oder mehr inerten festen und/oder flüssigen Träger-und/oder Hilfsstoff(en) gekennzeichnet sind.

Die erfindungsgemäßen Arzneimittel sind vorzugsweise solche mit anxiolytischer und/oder narkosepotenzierender Wirkung.

Ferner ist es bevorzugt, daß die erfindungsgemäßen Arzneimittel als Säureadditionssalz von 3-(Amino)-4-äthylthio)-chinolin 3-(Amino)-4-(äthylthio)-chinolin-hydrochlorid enthalten.

Die erfindungsgemäßen Arzneimittel können den beziehungsweise die Wirkstoff(e) 3-(Amino)-4-(äthylthio)-chinolin und/oder [ein] Säureadditionssalz(e) desselben in einer zur oralen Verabreichung geeigneten Form, zum Beispiel als Tabletten, überzogene Tabletten, Dragees, Weich-oder Hartgelatinekapseln, Lösungen, Emulsionen oder Suspensionen, in einer zur parenteralen Verabreichung geeigneter Form, zum Beispiel als injizierbare Lösungen, oder in einer zur rektalen Verabreichung geeigneten Form, zum Beispiel als Suppositorien, zubereitet enthalten.

Die erfindungsgemäßen Arzneimittel können als Arzneimittelpräparate vorliegen. Die erfindungsgemäßen Arzneimittelpräparate können nach an sich bekannten Verfahrensweisen der pharmazeutischen Technik durch Vermischen des beziehungsweise der Wirkstoffe[s] mit dem beziehungsweise den inerten festen und/oder flüssigen, organischen und/oder anorganischen pharmazeutischen Träger-und/oder Hilfsstoff(en) bereitet werden.

Bei der Herstellung der Tabletten, überzogenen Tabletten, Dragees und Hartgelaatinekapseln können als Träger zum Beispiel Milchzucker [Lactose], Maisstärke, Kartoffelstärke, Talk, Magnesiumcarbonat, Magnesiumstearat, Calciumcarbonat, Stearinsäure und/oder Salze derselben verwendet werden. Die Weichgelatinekapseln können als Träger zum Beispiel [ein] Pflanzenöl(e), Fett(e), Wachs(e) und/oder Polyol(e) geeigneter Konsistenz enthalten. Bei der Herstellung der Lösungen und Sirupe kann beziehungsweise können als Träger zum Beispiel Wasser, [ein] Polyol(e), Polyäthylenglykol(e), Rohrzucker [Saccharose] und/oder Glucose Verwendung finden. Die injizierbaren Lösungen können als Träger zum Beispiel Wasser, [einen] Alkohol(e), [ein] Polyol(e), Glycerin und/oder [ein] Pflanzenöl(e) enthalten. Bei der Herstellung von Suppositorien kann beziehungsweise können als Träger zum Beispiel [ein] Öl(e), Wachs(e), Fett(e) und/oder Polyol(e) geeigneter Konsistenz eingesetzt werden.

Die erfindungsgemäßen Arzneimittel können als Hilfsstoffe übliche, zum Beispiel 1 oder mehr Netz-, Dispergier-, Konservierungs-und/oder Emulgiermittel, Farbstoffe, Süßungsmittel, Aromastoffe, Salze zu Veränderung des osmotischen Druckes und/oder Puffer, enthalten. Ferner können sie 1 oder mehr weitere[n] pharmazeutisch wirksame[n] Stoff(e) enthalten.

Die Erfindung beinhaltet auch die Verwendung des 3-(Amino)-4-(äthylthio)-chinolines und/oder seiner Säureadditionssalze zur Herstellung von Arzneimitteln, insbesondere solchen mit anxiolytischer und/oder narkosepotenzierender Wirkung.

Das 3-(Amino)-4-(äthylthio)-chinolin der Formel I und/oder das beziehungsweise die Säureadditionssalz(e) desselben wird beziehungsweise werden vorzugsweise in Form von Tabletten oder Kapseln oral verabreicht. Tabletten und Kapseln mit einem Wirkstoffgehalt von 2,5 bis 50 mg haben sich als besonders vorteilhaft erwiesen. Die tägliche Dosis des 3-(Amino)-4-(äthylthio)-chinolines der Formel I beziehungsweise seiner Säureadditionssalze kann innerhalb weiter Grenzen variiert werden und hängt von mehreren Faktoren ab, zum Beispiel von der Wirksamkeit des Wirkstoffes, vom Zustand und Alter der Patienten und von der Schwere der Krankheit. Die tägliche orale Dosis beträgt im allgemeinen etwa 1 bis 300 mg und die tägliche parenterale Dosis liegt im allgemeinen im Bereich von etwa 0,5 bis 150 mg. Die obigen Bereiche sind nur informativen Charakters, wobei die tatsächliche Dosis auch oberhalb und unterhalb der angegebenen Grenzen liegen kann und immer vom Arzt zu bestimmen ist.

Die Erfindung wird an Hand der folgenden Beispiele näher erläutert.

Beispiel 1

17,62 (0,1 Mol) 3-(Amino)-4-(mercapto)-chinolin werden in 50 ml einer 2 molaren Natriumhydroxydlösung gelöst. Nach Zugabe von einigen Natriumpyrosulfitkristallen werden bei 60 °C 22 g (0,14 Mol)

Äthyljodid tropfenweise zugegeben. Das Reaktionsgemisch wird eine Stunde lang bei 60 °C gerührt. Das Produkt wird mit Chloroform extrahiert, der Extrakt mit Aktivkohle geklärt und eingedampft. Es werden 17,3 g 3-(Amino)-4-(äthylthio)-chinolin erhalten, Ausbeute 85%, Siedepunkt: 147 °C/26,6 $N.m^{-2}$

Die obige Base (gelbes Öl) wird in Äthylacetat mit chlorwasserstoffhaltigem Äthanol in das Hydrochlorid überführt. Es werden 20,0 g 3-(Amino)-4-(äthylthio)-chinolin-hydrochlorid erhalten, Ausbeute 98 %, F.: 228-230 °C (aus Methanol).

Beispiel 2

17,62 g (0,1 Mol) 3-(Amino)-4-(mercapto)-chinolin werden in 125 ml einer 2 molaren Natriumhydroxydlösung gelöst. Nach Zugabe von 23,30 g (0,125 Mol) Benzolsulfonsäureäthylester wird das Reaktionsgemisch 2 Stunden lang zum Sieden erhitzt. Das gebildete Produkt wird mit Chloroform extrahiert, dem Chloroformextrakt wird chlorwsserstoffhaltiges Äthanol zugesetzt. Es werdem 2,0 g 3-(Amino)-4-(äthylthio)-chinolin-hydrochlorid erhalten, Ausbeute 87 %, F.: 228-230 °C (aus Methanol).

Beispiel 3

20,52 g (0,1 Mol) 3-(Nitro)-4-(mercapto)-chinolin werden in 150 ml einer molaren Natriumhydroxydlösung aufgenommen. Nach Zugabe von einigen Natriumpyrosulfitkristallen werden bei 60 °C 23,3 g (0,15 Mol) Äthyljodid zugesetzt. Nach Beendigung der Reaktion wird das gebildete Produkt mit Dichlormethan extrahiert. Der Extrakt wird eingedampft. Das erhaltene 3-(Nitro)-4-(äthylthio)-chinolin (18,6 g) wird in Alkohol gelöst. Nach Zugabe einer wässrigen Natriumsulfidlösung wird das Reaktionsgemisch 2 Stunden lang unter Rückfluss erhitzt, mit Wasser verdünnt und mit Dichlormethan extrahiert. Der Extrakt wird geklärt und eingedampft. Ausbeute 83,5 % (13,6 g). Die so erhaltene Base wird in an sich bekannter Weise in das Hydrochlorid überführt. Es werden 14,0 g 3-(Amino)-4-(äthylthio)-chinolin-hydrochlorid erhalten. Das Produkt ist mit der nach den Beispielen 1 und 2 hergestellten Verbindung identisch.

Beispiel 4

Zu einer mit Chloroform gebildeten Lösung von 20,86 g (0,1 Mol) 3-(Nitro)-4-(chlor)-chinolin wird eine Lösung von 16,8 g Natriumäthanthiolat in einer Mischung von Methanol und Wasser bei 60 °C zugegeben. Das Reaktionsgemisch wird stehengelassen, wonach mit alkalischem Wasser mehrfach extrahiert und die Chloroformlösung eingeengt wird. Das so erhaltene 3-(Nitro)-4-(äthylthio)-chinolin wird auf die im Beispiel 3 bschriebene Weise reduziert und aufgearbeitet. Es wird das 3-(Amino)-4-(äthylthio)-chinolin-hydrochlorid erhalten.

Beispiel 5

Es werden Tabletten folgender Zusammensetzung hergestellt:

| Komponente | Menge, mg/Tablette |
|---|---|
| 3-(Amino)-4-(äthylthio)-chinolin-<br>-hydrochlorid | 25,0 |
| Maisstärke | 97,0 |
| Polyvinylpyrrolidon | 175,0 |
| Magnesiumstearat | 3,0 |
| Gesamtmenge: | 300,0 |

Das Gemisch des Wirkstoffes und der Maisstärke wird mit einer 10-15 gew.-%-igen wäßrigen Polyvinylpyrrolidonlösung befeuchtet, granuliert und bei 40-45 °C getrocknet. Die erhaltenen Granalien werden gründlich getrocknet, mit dem Magnesiumstearat vermischt und zu Tabletten geeigneter Grösse gepresst.

Beispiel 6

Es werden Kapseln folgender Zusammensetzung nach an sich bekannten Methoden der pharmazeutischen Industrie hergestellt:

| Komponente | Menge, mg/Kapsel |
|---|---|
| 3-(Amino)-4-(äthylthio)-chinolin-<br>-hydrochlorid | 20,0 |
| Milchzucker | 60,0 |
| Maisstärke | 17,0 |
| Talk | 2,0 |
| Magnesiumstearat | 1,0 |
| Gesamtgewicht: | 100,0 |

## Ansprüche

1.) 3-(Amino)-4-(äthylthio)-chinolin der Formel

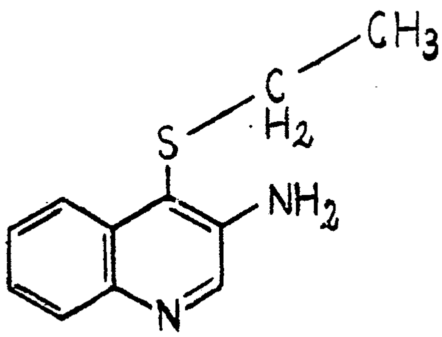

I

oder dessen Säureadditionssalze zur Verwendung als Arzneimittel.

2.) 3-(Amino)-4-(äthylthio)-chinolin oder dessen Säureadditionssalze zur Verwendung als Arzneimittel nach Anspruch 1, dadurch gekennzeichnet, daß das Arzneimittel ein solches mit anxiolytischer und/oder narkosepotenzierender Wirkung ist.

3.) Säureadditionssalz zur Verwendung als Arzneimittel nach Anspruch 1 oder 2, dadurch gekennzeichnet, daß es 3-(Amino)-4-(äthylthio)-chinolinhydrochlorid ist.

4.) Verfahren zur Herstellung des beziehungsweise der 3-(Amino)-4-(äthylthio)-chinolines beziehungsweise Säureadditionssalze desselben nach Anspruch 1 bis 3, dadurch gekennzeichnet, daß man

a) 3-(Amino)-4-(mercapto)-chinolin der Formel

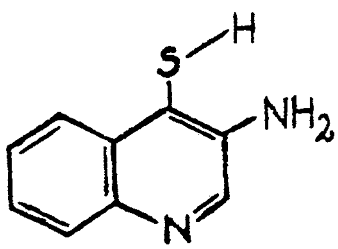

II

äthyliert oder

b) 3-(Nitro)-4-(mercapto)-chinolin der Formel

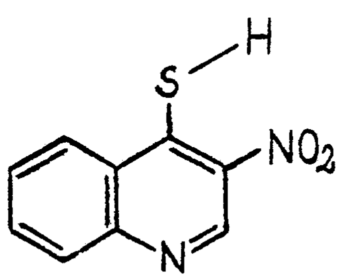

III

äthyliert und danach das erhaltene 3-(Nitro)-4-(äthylthio)-chinolin der Formel

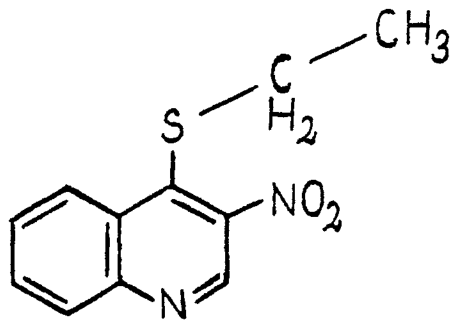

IV

reduziert oder
c) 3-(Nitro)-4-(chlor)-chinolin der Formel

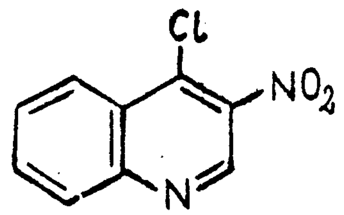

V

mit einem Alkaliäthanthiolat umsetzt und das erhaltene 3-(Nitro)-4-(äthylthio)-chinolin der Formel IV reduziert,
worauf man in an sich bekannter Weise gegebenenfalls das erhaltene 3-(Amino)-4-(äthylthio)-chinolin der Formel

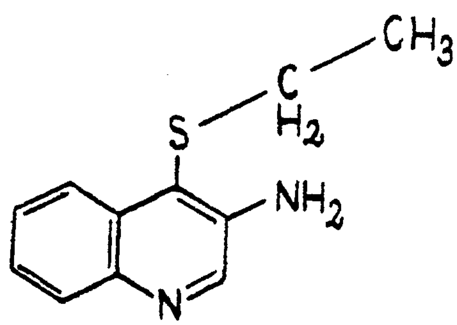

I

in ein Säureadditionssalz überführt und/oder das erhaltene Säureadditionssalz des 3-(Amino)-4-(äthylthio)-chinolines der Formel I in die freie 3-(Amino)-4-(äthylthio)-chinolinbase oder in ein anderes Säureadditionssalz überführt.

5.) Arzneimittel, gekennzeichnet durch einen Gehalt an 3-(Amino)-4-(äthylthio)-chinolin und/oder 1 oder mehr Säureadditionssalz(en) desselben als Wirkstoff(en) sowie 1 oder mehr inerten festen und/oder flüssigen Träger-und/oder Hilfsstoff(en).

6.) Arzneimittel nach Anspruch 5, dadurch gekennzeichnet, daß es solche mit anxiolytischer und/oder narkosepotenzierender Wirkung sind.

7.) Arzneimittel nach Anspruch 5 oder 6, dadurch gekennzeichnet, daß sie als Säureadditionssalz von 3-(Amino)-4-(äthylthio)-chinolin 3-(Amino)-4-(äthylthio)-chinolinhydrochlorid enthalten.

Patentsprüche für die folgenden Vertragsstaaten: GR, ES

1.) Verfahren zur Herstellung von 3-(Amino)-4-(äthylthio)-chinolin

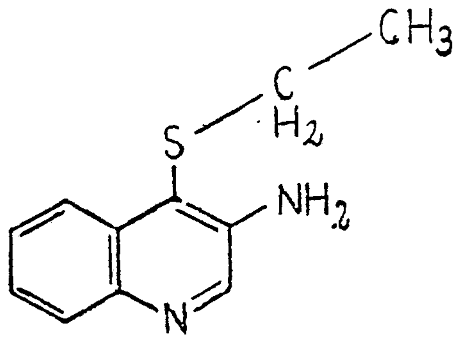

I

beziehungsweise Säureadditionssalzen desselben, dadurch gekennzeichnet, daß man a) 3-(Amino)-4-(mercapto)-chinolin der Formel

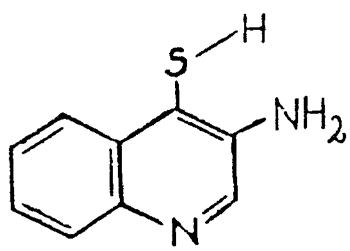

II

äthyliert oder

b) 3-(Nitro)-4-(mercapto)-chinolin der Formel

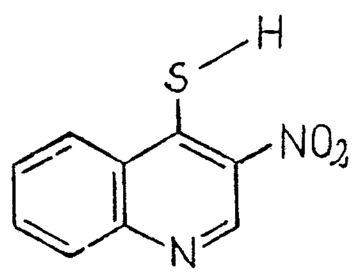

III

äthyliert und danach das erhaltene 3-(Nitro)-4-(äthylthio)-chinolin der Formel

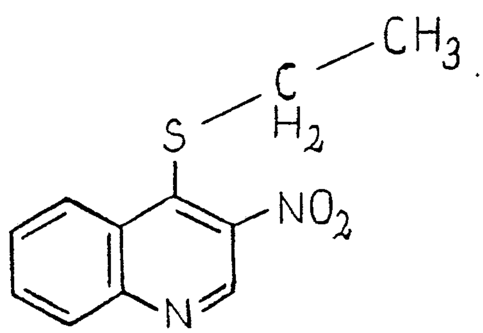

IV

reduziert oder

c) 3-(Amino)-4-(chlor)-chinolin der Formel

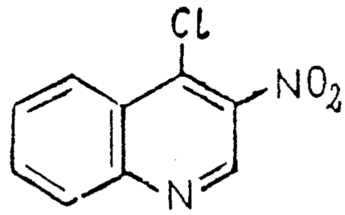

V

mit einem Alkaliäthanthiolat umsetzt und das erhaltene 3-(Nitro)-4-(äthylthio)-chinolin der Formel IV reduziert,
worauf man in an sich bekannter Weise gegebenenfalls das erhaltene 3-(Amino)-4-(äthylthio)-chinolin der Formel

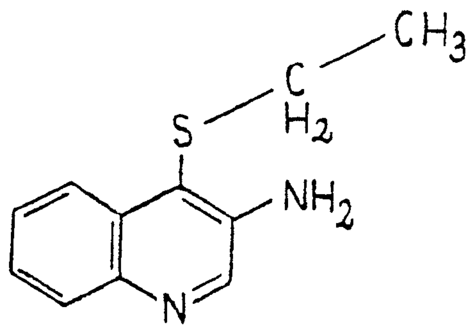

I

in ein Säureadditionssalz überführt und/oder das erhaltene Säureadditionssalz des 3-(Amino)-4-(äthylthio)-chinolines der Formel I in die freie 3-(Amino)-4-(äthylthio)-chinolinbase oder in ein anderes Säureadditionssalz überführt.

2.) Verfahren nach Anspruch 1, dadurch gekennzeichnet, daß man 3-(Amino)-4-(äthylthio)-chinolinhydrochlorid herstellt.